# EUROPEAN PATENT SPECIFICATION

(11) **EP 2 288 380 B1**
(45) Date of publication and mention of the grant of the patent: **03.06.2020**
(21) Application number: 09773063.4
(22) Date of filing: 09.06.2009
(51) Int. Cl.: A61K 39/00, C07K 14/005, C12N 7/00, A61K 39/29, A61K 39/12

(54) **VACCINE COMPOSITION USEFUL FOR HPV AND HEPATITIS B INFECTIONS AND A METHOD FOR PREPARING THE SAME**
IMPFSTOFFZUSAMMENSETZUNG FÜR HPV- UND HEPATITIS-B-INFEKTIONEN SOWIE VERFAHREN ZU IHRER HERSTELLUNG
COMPOSITION DE VACCIN UTILE POUR LES INFECTIONS PAR LE VIRUS DE L'HÉPATITE B ET PAR LE PAPILLOMAVIRUS HUMAIN ET SON PROCÉDÉ DE PRÉPARATION

(30) Priority: 09.06.2008 IN CH14042008
(43) Date of publication of application: 02.03.2011
(73) Proprietor: Bharat Biotech International Limited, Hyderabad 500 078 (IN)
(72) Inventor: ELLA, Krishna, Murthy, Hyderabad 500 078 (IN); SUMATHY, Kandaswamy, Hyderabad 500 078 (IN)
(74) Representative: dompatent von Kreisler Selting Werner - Partnerschaft von Patent- und Rechtsanwälten mbB
(86) International application number: PCT/IN2009/000333
(87) International publication number: WO 2010/001409

(56) References cited:
- WO-A1-2006/108226
- WO-A2-01/17550
- WO-A2-2007/095320
- US-A1- 2008 118 527
- TINDLE R W ET AL: "CHIMERIC HEPATITIS B CORE ANTIGEN PARTICLES CONTAINING B- AND TH-EPITOPES OF HUMAN PAPILLOMAVIRUS TYPE 16 E7 PROTEIN INDUCE SPECIFIC ANTIBODY AND T-HELPER RESPONSES IN IMMUNISED MICE" VIROLOGY, ACADEMIC PRESS,ORLANDO, US, vol. 200, 1 January 1994 (1994-01-01), pages 547-557, XP002074501 ISSN: 0042-6822
- BAEZ-ASTUA ANDRES ET AL: "Low-dose adenovirus vaccine encoding chimeric hepatitis B virus surface antigen-human papillomavirus type 16 E7 proteins induces enhanced E7-specific antibody and cytotoxic T-cell responses", JOURNAL OF VIROLOGY, THE AMERICAN SOCIETY FOR MICROBIOLOGY, US, vol. 79, no. 20, 1 October 2005 (2005-10-01), pages 12807-12817, XP002578882, ISSN: 0022-538X, DOI: 10.1128/JVI.79.20.12807-12817.2005

## Description

### FIELD OF THE INVENTION:

The present invention relates to antigenic formulations consisting of chimeric fusion antigens of HPV antigens with Hepatitis B surface antigen (HbsAg) and an adjuvant, wherein the HPV-HbsAg chimeric fusion antigens are selected from the group consisting of SEQ ID No. 1 and SEQ ID No. 2 as disclosed herein as well as to a method for preparing the HPV-HbsAG chimeric fusions.

The present invention relates further to a pharmaceutical composition for use in the prophylaxis and treatment against human papillomavirus and Hepatitis B infections comprising an antigenic formulation according to the invention and a pharmaceutically acceptable carrier.

### BACKGROUND OF THE INVENTION:

The Human papillomaviruses (HPVs) are double stranded DNA viruses that infect squamous and mucosal epithelia. The malignant epithelial neoplasms caused by the 'high-risk' or the oncogenic HPV types could progress to malignant cervical carcinoma (Bosch and de Sanjose, 2003). Persistent infection with HPV significantly correlates with progression to invasive carcinoma. The 'high risk' genotypes may progress differentially from LSIL (low grade squamous intraepithelial lesions) to malignancy (Schlecht et al. 2003). HPV is the major etiological factor for cervical cancer as almost 99.7 % of cervical cancer cases are found to have persistent HPV infections (Walboomers et al, 1999). The incidence of HPV infection is common among sexually active young women, and over 95% of the incident infections appear to resolve spontaneously over a period of time. Invasive cervical carcinoma develops in less than 5% of HPV16 infected women (Schlecht et al. 2003).

Of die nearly 100 types of HPV molecularly identified to date and more than HPV types identified in the genital tract, HPV 16 accounts for 50-60% of the cervical cancer cases in most countries, followed by HPV 18 (10-20%), and HPVs 31 and 45 (4-5% each). Thus, HPV16 is the most common type in all geographic regions with HPV18 following closely, particularly being more common in Southeast Asia. While this pattern is common in squamous cell carcinomas (SCCs) worldwide, HPV18 is most predominant in case of adenocarcinomas (ADCs) followed by HPV16 (Clifford et al 2003). Epidemiological studies have shown mixed infection of several HPV genotypes.

Evidence from other studies show that HPV16 is more persistent (Richardson et al. 2003; Londesborough et al. 1996), as well as and more likely to progress to CIN3 than other high-risk HPV genotypes. Thus, in HPV-based cervical screening programs, genotyping may have diagnostic utility in separating LSIL cases positive for HPV16 (and perhaps HPV18 and other genotypes), who require the closest surveillance, from LSIL cases infected with other high-risk genotypes (Clifford et al. 2005).

Vaccination strategies based on available epidemiological data on the age and type specific distribution of HPV infection could form the basis of developing an effective vaccine for controlling and eradicating human papillomavirus infection. Candidate vaccines that confer broad protective efficacy against multiple genotypes should be the strategy to consider for effective vaccination. The available vaccines using the L1 capsid proteins are type specific for HPV16 HPV18, HPV6 and HPV11 and offer inadequate protection to infection caused by other genotypes such as HPV31, HPV35, HPV52, HPV58 etc. which are also associated with progression to cervical carcinoma. There are no HPV vaccines available as yet, that offers cross protection against multiple HPV genotypes.

Two preventive vaccines comprising recombinant HPV L1 virus-like particles (VLPs) have been licensed. They target only two of the approximately 15 known oncogenic HPV types. Although approximately 70% of cervical cancer cases are attributed to these two types and there is evidence for some degree of Ciption of tross-protection against other closely related types. However, the current cost of these vaccines precludes sustained global delivery particularly in third world countries where the incidence of HPV infection is high.

Robert W. Tindle et al. (Chimeric Hepatitis B Core Antigen Particles containing Band Th-Epitopes of Human Papillomavirus Type 16E7 protein Induce Specific Antibody and T-helper Responses in Immunised Mice, Virology 200, 547-557 (1994)) discloses the use of Hepatitis B core antigen as an immunogenic delivery vehicle for foreign epitopes, in particular T-helper epitope of HPV-type 16E7.

WO 01/17550 A2 discloses combined vaccine compositions comprising a Hepatits B viral antigen and a HPV antigen. The vaccine compositions are formulated with an adjuvant which is a preferential stimulator Th1-cell response.

A. Bàez-Astúa et al. (Low-Dose Adenovirus Vaccine Encoding Chimeric Hepatitis B Virus Surface Antigen-Human Papillomavirus Type 16 E7 Proteins Induces Enhanced E7-specific Antibody and Cytotoxic T-Cell responses; Journal of Virology, Vol 69, No. 20, p. 12807-12817 (Oct. 2005)) suggests vaccines based on chimeric HbsAg(S) carrying C-terminally fused E7 as an effective candidate for immunotherapy for cervical cancers caused by HPV-16 and immunization against HBV.

### BRIEF DESCRIPTION OF THE DRAWINGS:

Fig.1: depicts PCR amplification of Hepatitis B surface antigen (HbsAg) gene. Lane 1: 700 bp HbsAg gene; Lane 2: 100 bp DNA ladder
Fig.2: depicts PCR amplification of the multiepitope sequences of E6 and E7 synthetic gene fragment for construction of chimeric HbsAg-HPV chimera. Lanel: ∼ 150 bp multiepitope gene fragment consisting of the 129 bp of the multiepitope sequence; Lane 2: 100 bp DNA ladder

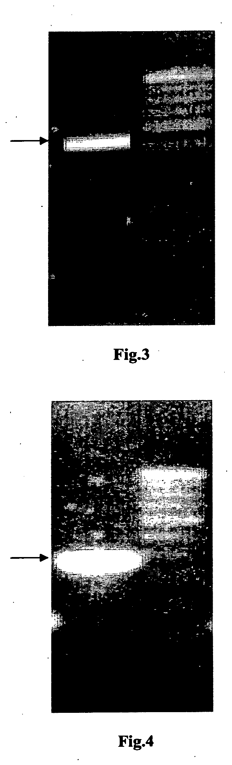

Fig.3: depicts PCR amplification HPV16 L2 ∼ 530 bp gene fragment consisting of 510 bp of the HPV16 L2 gene for generation of chimeric HbsAg-HPV sequence.; Lane 2: 100 bp DNA ladder
Fig.4: depicts PCR amplification of the HPV16 L2 fragment corresponding to amino acids 100-220 of the L2 gene to obtain ∼ 380 bp PCR fragment that contains 360 bp of the specific sequence

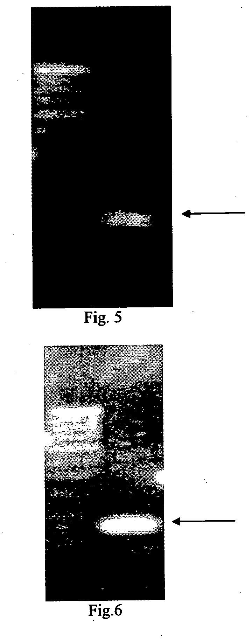

Fig. 5: illustrates PCR amplification of the HPV16 L1 gene fragment of 99bp amplified by PCR to obtain a ∼ 120 bp fragment. corresponding to amino acids 100-220 of the L2 gene to obtain ∼ 380 bp PCR fragment that contains 360 bp of the specific sequence
Fig. 6: illustrates PCR amplification of HPV18 L1 gene gives a ∼ 140 bp fragment consisting of 117 bp of the HPV18 L1 sequence

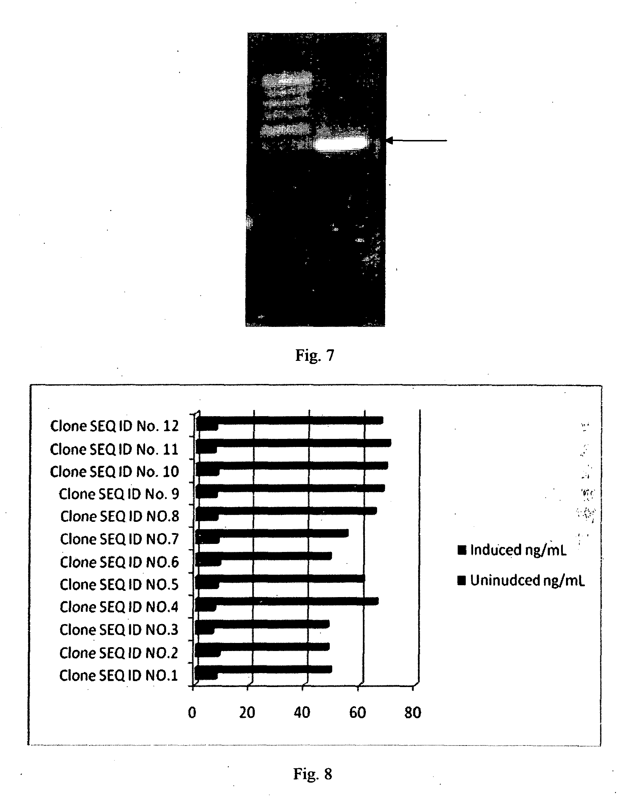

Fig. 7: depicts PCR amplification of HPV18 L1 gene gives a ∼500 bp fragment consisting of 480 bp of HPV18 L1 fragment.
Fig. 8: shows the expression level of the chimeric HbsAg -HPV protein detected for HbsAg content by ELISA and taken as a measure of the expression of the chimeric protein in the induction cultures of *Pichia pastoris*

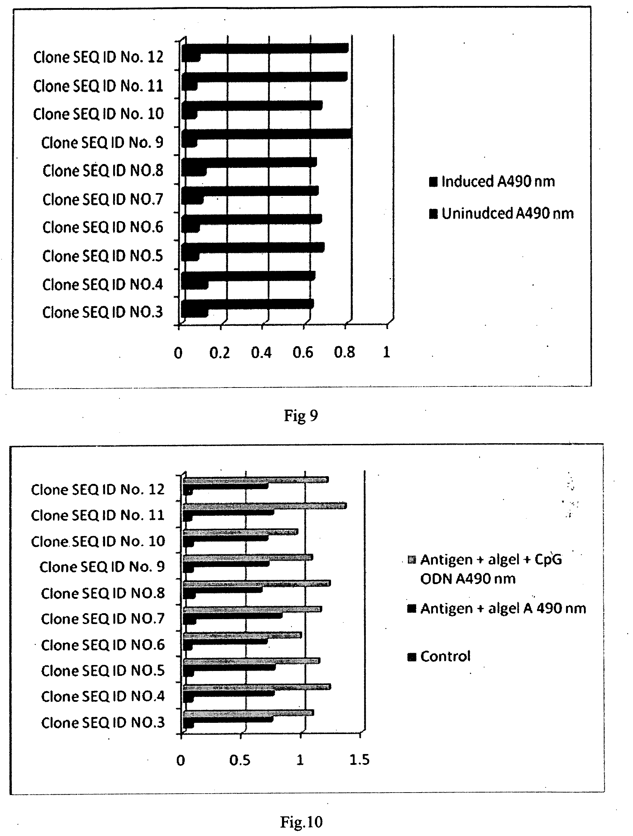

Fig. 9: relates to the expression level of the chimeric HbsAg -HPV protein detected for HPV antigen content by ELISA in the induction cultures of *Pichia pastoris*
Fig.10: Immunogenicity of the chimeric proteins detected using HPV specific ELISA using primary antibody raised against the commercially available tetravalent vaccine and for HPV16 L2 die rabbit antisera raised against the purified protein.

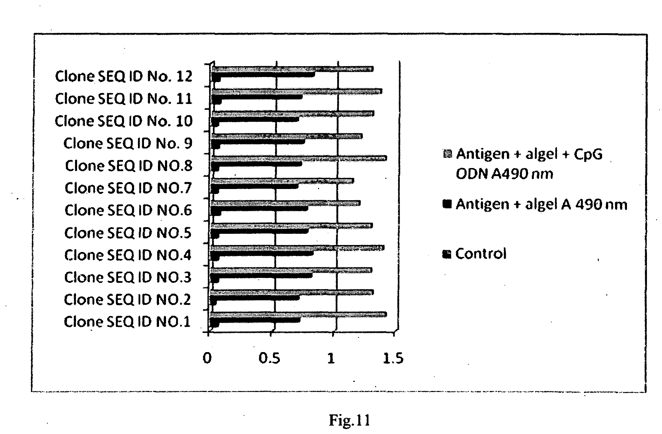

Fig.11: Immunogenicity of die chimeric proteins detected using HbsAg specific ELISA using die commercially available kit. The antibody titer against the construct containing HPV 16 L2 protein was estimated using the rabbit antisera raised against the purified L2 protein.

### SUMMARY OF THE INVENTION:

The present invention provides an antigenic formulation according to claim 1, a method for preparing said HPV-HbsAg chimeric fusions according to claim 3, and a pharmaceutical composition comprising said antigenic formulation as defined in claim 4.

Accordingly, the present specification provides a vaccine composition comprising chimeric fusions of the HPV antigens with viral or bacterial proteins conferring enhanced immunogenicity useful for Hepatitis B virus as well as human papillomavirus (HPV) infections.

The HPV antigens may be selected from a group comprising but not limited to HPV16, HPV18, HPV6, HPV11, HPV31, HPV33, HPV35, HPV39, HPV45, HPV51, HPV52, HPV56, HPV58, HPV59, HPV68.

The antigenic composition comprising of complete/partial sequences /mutants /variants of the HPV antigens may be selected from the list comprising L1, L2, E2, E5, E6 and E7 antigens or specific T cell or B cell epitopes of the said antigens either as single sequence or in tandem repeats in chimeric fusions with bacterial/viral proteins capable of inducing a strong antibody response when administered to the host.

The viral and bacterial proteins may be selected from a list including but not limited to hepatitis B antigens such as hepatitis B core antigen, hepatitis B surface antigen, inactivated tetanus toxoid, diphtheria toxoid, *E.coli* heat labile toxin, cholera toxin, *Pseudomonas* exotoxin A, Shiga toxin, listerolysin either singly or in combinations or epitopes derived from the aforementioned proteins thereof.

The antigenic composition consists of chimeric fusions of HPV antigens with hepatitis B surface antigen (HbsAg).

The HPV antigen comprises either the complete or partial sequences/epitopes of the capsid proteins such as L1 and L2 or polypeptides derived from L1 and/or L2 thereof and fused to the HbsAg such as in SEQ ID NO. 3 to SEQ ID NO. 13.

The HPV antigens comprises complete / partial sequence / mutants /variants / epitopes of the HPV early proteins such as E2, E5, E6 and E7 either alone or in combinations thereof such as in SEQ ID No. 1 and 2.

The HPV antigens are fused either to the N-terminus or C-terminus or at any region within the open reading frame of the hepatitis B surface antigen with or without the addition of linker sequence between the two sequences.

According to another aspect of this specification there is provided a Method for expressing the afore mentioned HPV and HPV-HBsAg chimeric antigens of claim 1 comprising cultivating host cell transformed with antigen/ fusion antigen of claim 1 isolating and purifying the recombinant proteins there from.

The recombinant expression of the afore mentioned HPV antigens may be either as intracellular proteins or as secretory proteins in the host, the host being either prokaryotic or eukaryotic expression system preferably yeast.

A pharmaceutical composition comprising antigenic compositions as disclosed herein before in an effective amount to be used as vaccine and pharmaceutically acceptable carrier.

The antigens are used in the range of 10 µg - 1000 µg of the protein per dose, preferably between 10 µg to 200 µg and most preferably between 10 µg to 100 µg.

The pharmaceutical composition further comprising a suitable adjuvant, the adjuvant being selected from at least one of the following: aluminium hydroxide, aluminium phosphate, monophosphoryl lipid A, other organic lipophilic compounds, CpG and non-CpG containing oligonucleotides, cytokines.

A Method of eliciting protective antibody response to antigenic composition and/or cytotoxic T cell response according to any preceding claims comprising administering the said composition to the subject that is in need for such treatment by at least one of the routes such as oral, mucosal or parenteral routes of administration.

Aforementioned antigenic compositions is effected in pharmaceutically acceptable biodegradable polymers.

The use of the aforementioned antigenic formulations for prophylaxis or therapeutic treatment of papillomavirus infections and associated risk of cancer in mammals particularly in humans.

### DETAILED DESCRIPTION OF THE INVENTION:

The invention covers the development of prophylactic and therapeutic vaccine candidates for human papillomavirus infections in humans. It is known in the prior art that the major capsid protein of HPV, the L1 protein can assemble into Virus Like Particle (VLP) either alone or in combination with the minor capsid protein L2, when expressed as recombinant proteins in vitro. These virus-like particles are immunogenic and have been used as vaccines for prophylaxis of HPV infections in humans Cervical carcinoma is caused by several 'oncogenic' HPV types including HPV16, HPV18, HPV31, HPV33, HPV35, HPV39, HPV45, HPV51, HPV52, HPV56, HPV58, HPV59, HPV68 etc. VLPs comprising the L1 capsid proteins are good vaccine candidates but the antibodies that recognize specific structural epitopes on the VLP are genotype specific and the antibodies against one VLP type shows little or no cross reactivity against the other HPV genotypes. However, mixed infection of several HPV genotypes is common.

The scope of the present invention is to develop a broad based vaccine that would offer protective immunity against the major HPV types that are associated with the risk of cervical carcinoma. The minor HPV capsid protein L2 potentially offers a broad neutralizing activity against several genotypes of the virus by virtue of higher sequence conservation in the L2 protein. However, unlike the structural epitopes of L1 that are highly antigenic, L2 protein is relatively weakly immunogenic. Its low abundance in natural capsids- about 12-72 molecules per 360 copies of L1 limits its immunogenicity (Pereira et al. 2009) A possible approach to broader immunity at lower cost is to consider vaccination against L2. L2 vaccines can be produced inexpensively and they also have the promise of conferring much broader cross-type protective immunity than that-observed with L1 VLP immunization (Kanda and Kondo 2009). The minor capsid protein L2 of HPV that plays important roles in virus entry into cells, localization of viral components to the nucleus, in DNA binding, capsid formation and stability elicits antibodies that are more cross-reactive between HPV types than does the major capsid protein L1, making it an attractive potential target for new-generation, more broadly protective subunit vaccines against HPV infections.

One object of the invention relates to increasing the immunogenicity of the candidate vaccine(s) by allowing presentation of multiple copies of the antigenic epitopes for enhancing immunogenicity by chimeric fusion with viral and bacterial proteins. So far no multi-epitope vaccine for HPV infection has been commercialized. Multi-epitope vaccines offer the advantage of broad protective activity by virtue of high sequence conservation amongst the antigenic epitopes of the capsid and early proteins across different genotypes of the virus.

The virus like particles formed by hepatitis B surface antigen (HbsAg) is a multimeric structure that is highly immunogenic when administered as a vaccine against hepatitis B virus infection. The structure of the VLP allows invention of heterologous sequences from other proteins into its open reading frame at discrete locations allowing the use of chimeric fusions of proteins with HbsAg to be used as potential vaccine candidates. The HPV antigens include the capsid proteins L1 and L2, and the early proteins such as E1, E2, E5, E6 and E7. These proteins could consist of complete / partial sequence / mutants / variants / epitopes of the HPV L1, L2, E1, E2, E5, E6 and E7 proteins either singly or in combinations thereof. The T cell or B cell epitopes of these candidate proteins are used either singly or in tandem repeats and fused to the HbsAg are used as vaccine candidates for HPV vaccination. The HPV antigens are fused either to the N-terminus or C-terminus or at any region within the open reading frame of the hepatitis B surface antigen with or without the addition of linker sequence between the two antigens.

Chimeric fusion of the HPV capsid proteins to HbsAg has not been reported so far. Chimera of the minor HPV capsid protein L2 with HbsAg is attractive as vaccine candidate as the L2 protein although offering a broad neutralizing activity against multiple genotypes of the virus, is by itself a weak antigen as it is a linear polypeptide. Representation of the L2 regions responsible for its broad neutralizing activity against the multiple genotypes of the HPV virus as multimeric copies on the VLP of HbsAg increases its immunogenicity by virtue of representation of multiple copies on the VLP than when presented as a single copy. The regions of the HPV16 and HPV18 L1 proteins chosen for making die chimeric constructs with HbsAg also are those that have a broad neutralizing activity against other genotypes and could potentially offer cross-protection. We have exploited the flexibility of HbsAg whose self-assembly into virus like particles still allows chimeric fusion with heterologous sequences with other proteins. We have made a chimera of HbsAg with B cell and T cell epitopes of HPV E6 and E7 proteins that are presented in tandem to increase their immunogenic potential as well as with HPV16 and HPV18 L1 and L2 proteins. Chimeric construct of discrete regions of HPV E7 with HbsAg protein has been reported. We have used multiple T and B cell epitopes derived from E6 and E7 proteins of HPV as presence of these sequences in tandem elicit higher immunogenicity than when present as a single copy. Their presentation on the multimeric VLP of HbsAg in turn increases die number of copies that are displayed on the VLP which further increases their immunogenicity and represents an excellent vaccine candidate. The B and T cell epitopes are known to elicit strong cytotoxic T cell responses offering die promise of a good therapeutic vaccine against HPV infections.

Those skilled in the art would identify that the following candidates could also be used in a similar strategy as chimeric sequences with HPV antigens in order to increase their vaccine potential. The list of candidates includes but is not limited to: hepatitis B core antigen, inactivated tetanus toxoid, diphtheria toxoid, *E.coli* heat labile toxin, cholera toxin, *Pseudomonas* exotoxin A, Shiga toxin, listerolysin, heat shock proteins, calreticulin or epitopes derived from the aforementioned proteins thereof.

The abovementioned proteins can be expressed in either prokaryotic or eukaryotic expression systems as intracellular or secretory proteins. *Pichia pastoris* as an expression system is advantageous at industrial scale as it is cost effective and few proteins that have expressed and purified from *Pichia pastoris* have been successfully commercialized as it has been found safe for human use. This system would be applicable to yeast of any genus and species. The chimeric fusion of the HPV antigens with secretory signal of the HSA (human serum albumin) to increase the synthesis and secretion of the protein into the culture medium enables not only high level of expression but also makes it easy to purify the protein at the scale of industrial fermentation. The sequence of HSA at its N-terminus is compatible with high level of synthesis and efficient secretion in *Pichia pastoris.* Purification of the virus antigens using the proprietary Himax™ technology facilitates isolation of the HbsAg-HPV chimeric antigens particularly VLPs in a cost-effective method that is safe for human application and economical at the industrial scale than conventional density gradient ultracentrifugation on cesium chloride. This will enable the production of low cost vaccine and is environmental friendly to avoid the use of heavy metals like cesium chloride.

The proteins are expressed either as VLPs, capsomeres, polypeptides or chimeric polypeptides comprising HPV and the bacterial/viral antigens. The said antigens incorporated either as single sequence or in tandem repeats as chimeric fusions with bacteriail/viral proteins capable of inducing a strong antibody response or a cytotoxic T cell response when administered to the host. Subunit immunogens containing copies of T- and B-cell epitopes in tandem are potentially more immunogenic than when they occur as a single copy. The chimeric constructs with E6 and E7 epitopes as well with L2 and L1 proteins have elicited a strong antibody response against both the HbsAg and the HPV epitopes as determined by ELISA. This offers a vaccine for dual use, against both Hepatitis B and as well as with multiple genotypes of the HPV virus.

The antigenic compositions of the abovementioned proteins are formulated in pharmaceutically acceptable carrier for immunization in humans. The antigenic formulations can be administered either alone or with an adjuvant selected from a list that includes but is not limited to: aluminium hydroxide, aluminium phosphate, monophosphoryl lipid A, other organic lipophilic compounds, CpG and non-CpG containing oligonucleotides, cytokines etc. Alternatively, the antigenic formulation could comprise two or more purified antigens that are mixed in vitro in a suitable formulation and used as a vaccine. The antigenic formulations can be delivered by one of the several methods either by oral, mucosal or by parenteral routes in mammals preferably in human subjects' in order to elicit a protective antibody and/or cytotoxic T cell response. The antigens could also be delivered in pharmaceutically acceptable biodegradable polymers. The aforementioned formulations could be used for prophylaxis or therapeutic treatment of papillomavirus infections and associated risk of cancer in mammals.

HPV enters the body through mucosal cells and does not spread systemically. Therefore, an HPV vaccine will possibly have to induce a strong and sustained immune response at the genital mucosa. In this respect, nasal and oral immunization studier in animals have shown VLPs to induce antibodies in genital mucosa. Vaccines conferring mucosal immunity against HPV antigens are advantageous for prophylaxis of HPV infection. Formulations suitable for mucosal delivery of the vaccines would be tested. No such HPV vaccine is commercially available for mucosal application.

### Examples:

### 1. Example 1: Cloning Strategy

### a) Strategy 1: Chimeric fusion of the multiepitopes derived from early proteins to HbsAg:

This sequence consists of the hepatitis B surface antigen fused to the multiepitope derived from the HPV16 and HPV18 E7 proteins that are in tandem, followed by chimeric fusion of the multiepitope to the C-terminus and N-terminus of the HbsAg protein. HbsAg protein sequence:

MENTTSGFLGPLLVLQAGFFLLTRILTIPQSLDSWWTSLNFLGGAPACPGQNSQSP
TSNHSPTSCPPICPGYRWMCLRRFIIFLFILLLCLISLLVLLDYQGMLPVCPPLLGKS
NTTTTSTGPCKTCTMPAQGTSMFPSCCCTKPSDGNCTCIPIPSSWAFARFLWEWA
SVRFSWLSLLVPFVQWFVGLSPTVWLSVIWMMWYWGPSLYNILSPFLPLLPISCC
LWAYI.

The following epitopes derived from the E7 and/or E6 proteins are arranged in tandem to obtain: 5' EYMLDLQPETTEEDEIDGPAGQAEPDRAHYNIDEIDGVNHQHL 3' - 43 amino acids (129 nucleotides). This major epitope is a combination of the following and is selected from the list of T and B cell epitopes: HPV16 E7 T cell epitope: aa 20 - 29: TDLYCYEQLN; aa 45 - 54 AEPDRAHYNI; B CELL EPITOPES: aa 10 - 20: EYMLDLQPETT; aa 35 - 49 EEDEIDGPAGQAEPDR; fused to a HPV18 E7 B cell epitope DEIDGVNHQHL which is an immunodominant epitope of HPV18 E7 inorder to the obtain the following chimeric fusion designated as SEQ ID No. 1. A similar chimeric fusion of the multiepitope fused at the N-terminus of the HbsAg is the sequence provided in SEQ ID No.2. An initiator methionine for translation was introduced at the 5' end of the multiepitope for initiation of translation.

SEQ ID No.1
MENTTSGFLGPLLVLQAGFFLLTRILTIPQSLDSWWTSLNFLGGAPACPGQNSQSPTSNHSP
TSCPPICPGYRWMCLRRFIIFLFILLLCLISLLVLLDYQGMLPVCPPLLGKSNTTTTSTGPC
KTCTMPAQGTSMFPSCCCTKPSDGNCTCIPIPSSWAFARFLWEWASVRFSWLSLLVPFVQWF
VGLSPTVWLSVIWMMWYWGPSLYNILSPFLPLLPISCCLWAYIGSEYMLDLQPETTEEDEID
GPAGQAEPDRAHYNIDEIDGVNHQHL-

SEQ ID NO.2:

MEYMLDLQPETTEEDEIDGPAGQAEPDRAHYNIDEIDGVNHQHLGSENTTSGFLGPLLVLQA
GFFLLTRILTIPQSLDSWWTSLNFLGGAPACPGQNSQSPTSNHSPTSCPPICPGYRWMCLRR
FIIFLFILLLCLISLLVLLDYQGMLPVCPPLLGKSNTTTTSTGPCKTCTMPAQGTSMFPSCC
CTKPSDGNCTCIPIPSSWAFARFLWEWASVRFSWLSLLVPFVQWFVGLSPTVWLSVIWMMWY
WGPSLYNILSPFLPLLPISCCLWAYI-

### b) Strategy 2: Chimeric fusion of the minor capsid protein L2 to HbsAg.

The N-terminal region of the minor capsid protein L2 was fused to the HbsAg at the C-terminus of the HbsAg. The regions of L2 amplified are 171 amino acids of the following sequence (initiator methionine was introduced to facilitate translation) fused at the C-terminus of the HbsAg

MSMGVFFGGLGIGTGSGTGGRTGYIPLGTRPPTATDTLAPVRPPLTVDPVGPSDPSIVSLVE
ETSFIDAGAPTPVPSIPPDVSGFSITTSTDTTPAILDINNTVTTVTTHNNPTFTDPSVLQPP
TPAETGGHFTLSSSTISTHNYEEIPMDTFIVSTNPNTVTSSTPIPGSR

SEQ ID N0. 3:
MENTTSGFLGPLLVLQAGFFLLTRILTIPQSLDSWWTSLNFLGGAPACPGQNSQSPTSNHSP
TSCPPICPGYRWMCLRRFIIFLFILLLCLISLLVLLDYQGMLPVCPPLLGKSNTTTTSTGPC
KTCTMPAQGTSMFPSCCCTKPSDGNCTCIPIPSSWAFARFLWEWASVRFSWLSLLVPFVQWF
VGLSPTVWLSVIWMMWYWGPSLYNILSPFLPLLPISCCLWAYIGSSMGVFFGGLGIGTGSGT
GGRTGYIPLGTRPPTATDTLAPVRPPLTVDPVGPSDPSIVSLVEETSFIDAGAPTPVPSIPPDV
SGFSITTSTDTTPAILDINNTVTTVTTHNNPTFTDPSVLQPPTPAETGGHFTLSSSTISTHNYEEIPMD
TFIVSTNPNTVTSSTPIPGSR-

### SEQ ID N0. 4:

The minor capsid protein sequence L2 comprising 171 amino acids of the following sequence:
is fused at its C-terminus to the N-terminus of HbsAg to obtain SEQ ID N0.4. An initiator methionine has been added for translation.

5′
MSMGVFFGGLGIGTGSGTGGRTGYIPLGTRPPTATDTLAPVRPPLTVDPVGPSDPSIVSLVE
ETSFIDAGAPTPVPSIPPDVSGFSITTSTDTTPAILDINNTVTTVTTHNNPTFTDPSVLQPPTPAETG
GHFTLSSSTISTHNYEEIPMDTFIVSTNPNTVTSSTPIPGSRGSENTTSGFLGPLLVLQAGFFLLT
RILTIPQSLDSWWTSLNFLGGAPACPGQNSQSPTSNHSPTSCPPICPGYRWMCLRRFIIFLF
ILLLCLISLLVLLDYQGMLPVCPPLLGKSNTTTTSTGPCKTCTMPAQGTSMFPSCCCTKPSD
GNCTCIPIPSSWAFARFLWEWASVRFSWLSLLVPFVQWFVGLSPTVWLSVIWMMWYWGPSLY
NILSPFLPLLPISCCLWAYI 3′

### c) Strategy No.3:

### Chimeric fusion of the nested deletions of the L2 fragment to HbsAg.

The N-terminal region of the minor capsid protein L2 was fused to HbsAg at the C-terminus of the HbsAg. The region of L2 amplified is about 120 aa (360 bp) of the following sequence:

SDPSIVSLVEETSFIDAGAPTPVPSIPPDVSGFSITTSTDTTPAILDINNTVTTVTTHNNPT
FTDPSVLQPPTPAETGGHFTLSSSTISTHNYEEIPMDTFIVSTNPNTVTSSTPIPGSR

It is ligated to the C-terminus of the HbsAg to obtain SEQ ID No.5

SEQ ID No.5:

5′
MENTTSGFLGPLLVLQAGFFLLTRILTIPQSLDSWWTSLNFLGGAPACPGQNSQSPTSNHSP
TSCPPICPGYRWMCLRRFIIFLFILLLCLISLLVLLDYQGMLPVCPPLLGKSNTTTTSTGPC
KTCTMPAQGTSMFPSCCCTKPSDGNCTCIPIPSSWAFARFLWEWASVRFSWLSLLVPFVQWF
VGLSPTVWLSVIWMMWYWGPSLYNILSPFLPLLPISCCLWAYIGSSDPSIVSLVEETSFIDA
GAPTPVPSIPPDVSGFSITTSTDTTPAILDINNTVTTVTTHNNPTFTDPSVLQPPTPAETGG
HFTLSSSTISTHNYEEIPMDTFIVSTNPNTVTSSTPIPGSR 3′

### SEO ID No. 6:

The minor capsid protein sequence L2 comprising the following sequence of 120 amino acids: 
SDPSIVSLVEETSFIDAGAPTPVPSIPPDVSGFSITTSTDTTPAILDINNTVTTVTTHNNPT
FTDPSVLQPPTPAETGGHFTLSSSTISTHNYEEIPMDTFIVSTNPNTVTSSTPIPGSR
 is chimerically fused at its C-terminus to the N-terminus of HbsAg to Obtain SEQ ID NO.6 An initiator methionine was added for translation.

MSDPSIVSLVEETSFIDAGAPTPVPSIPPDVSGFSITTSTDTTPAILDINNTVTTVTTHNNP
TFTDPSVLQPPTPAETGGHFTLSSSTISTHNYEEIPMDTFIVSTNPNTVTSSTPIPGSRGSE

NTTSGFLGPLLVLQAGFFLLTRILTIPQSLDSWWTSLNFLGGAPACPGQNSQSPTSNHSPTS
CPPICPGYRWMCLRRFIIFLFILLLCLISLLVLLDYQGMLPVCPPLLGKSNTTTTSTGPCKT
CTMPAQGTSMFPSCCCTKPSDGNCTCIPIPSSWAFARFLWEWASVRFSWLSLLVPFVQWFVG
LSPTVWLSVIWMMWYWGPSLYNILSPFLPLLPISCCLWAYI 3'

### d) Strategy No. 4: Chimeric fusion of the HPV16 L1 region with HbsAg:

Chimeric fusion of L1 region of HPV16 protein at the C-terminus of HbsAg consisting of 33 amino acids derived from the L1 region of the HPV16 protein (99 bp) of the sequence: NTVIQDGDMVDTGFGAMDFITLQANKSEVPLDI

SEQ ID No.7

5′
MENTTSGFLGPLLVLQAGFFLLTRILTIPQSLDSWWTSLNFLGGAPACPGQNSQSPTSNHSPTSCPPIC
PGYRWMCLRRFIIFLFILLLCLISLLVLLDYQGMLPVCPPLLGKSNTTTSTGPCKTCTMPAQGTSMFP
SCCCTKPSDGNCTCIPIPSSWAFARFLWEWASVRFSWLSLLVPFVQWFVGLSPTVWLSVIWMMWYWGPS
LYNILSPFLPLLPISCCLWAYIGS NTVIQDGDMVDTGFGAMDFTTLQANKSEVPLDI 3′

Chimeric fusion of L1 region of HPV16 protein consisting of 33 amino acids derived from the L1 region of the HPV16 of the sequence: NTVIQDGDMVDTGFGAMDFTTLQANKSEVPLDI to the N-terminus of HbsAg in order to obtain the chimeric sequence of SEQ ID No. 8. An initiator methionine has been added for translation.

SEQ ID No. 8:

5′MNTVIQDGDMVDTGFGAMDFTTLQANKSEVPLDIGSENTTSGFLGPLLVLQAGFFLLTRILTIPQSL
DSWWTSLNFLGGAPACPGQNSQSPTSNHSPTSCPPICPGYRWMCLRRFIIFLFILLLCLISLLVLLDYQ
GMLPVCPPLLGKSNTTTTSTGPCKTCTMPAQGTSMFPSCCCTKPSDGNCTCIPIPSSWAFARFLWEWAS
VRFSWLSLLVPFVQWFVGLSPTVWLSVIWMMWYWGPSLYNILSPFLPLLPISCCLWAYI 3′

### e) Strategy No. 5: Chimeric fusion of HPV18 L1 region to HbsAg:

Chimeric fusion of the region from HPV18 L1 protein (39 amino acids; 117 bp) of the following sequence
5' PPLELKNTVLEDGDMVDTGYGAMDFSTLQDTKCEVPLDI 3'

Fused to the C-terminus of the HbsAg sequence to obtain the following chimeric sequence:

SEQ ID NO.9:

5′
MENTTSGFLGPLLVLQAGFFLLTRILTIPQSLDSWWTSLNFLGGAPACPGQNSQSPTSNHSP
TSCPPICPGYRWMCLRRFIIFLFILLLCLISLLVLLDYQGMLPVCPPLLGKSNTTTTSTGPC
KTCTMPAQGTSMFPSCCCTKPSDGNCTCIPIPSSWAFARFLWEWASVRFSWLSLLVPFVQWF
VGLSPTVWLSVIWMMWYWGPSLYNILSPFLPLLPISCCLWAYIGSPPLELKNTVLEDGDMVD
TGYGAMDFSTLQDTKCEVPLDI 3′

It is fused to the N-terminus of HbsAg protein to obtain the SEQ ID NO.10 where an initiator methionine has been added for translation.

SEQ ID NO.10:

5′
MPPLELKNTVLEDGDMVDTGYGAMDFSTLQDTKCEVPLDIGSENTTSGFLGPLLVLQAGFFL
LTRILTIPQSLDSWWTSLNFLGGAPACPGQNSQSPTSNHSPTSCPPICPGYRWMCLRRFIIF
LFILLLCLISLLVLLDYQGMLPVCPPLLGKSNTTTTSTGPCKTCTMPAQGTSMFPSCCCTKP
SDGNCTCIPIPSSWAFARFLWEWASVRFSWLSLLVPFVQWFVGLSPTVWLSVIWMMWYWGPS
LYNILSPFLPLLPISCCLWAYI

### f) Strategy No. 6: Chimeric fusion of a larger polypeptide of HPV18 L1 region to HbsAg:

Chimeric fusion of a larger fragment of HPV18 consisting of the following sequence 
ATSNVSEDVRDNVSVDYKQTQLCILGCAPAIGEHWAKGTACKSRPLSQGDCPPLELKNTVLE
DGDMVDTGYGAMDFSTLQDTKCEVPLDICQSICKYPDYLQMSADPYGDSMFFCLRREQLFAR
HFWNRAGTMGDTVPQSLYIKGTGMRASPGSCVYSPS
 and fused at its N-terminus to the C-terminus of the HbsAg to obtain the chimeric sequence:

SEQ ID NO. 11:

5′
MENTTSGFLGPLLVLQAGFFLLTRILTIPQSLDSWWTSLNFLGGAPACPGQNSQSPTSNHSP
TSCPPICPGYRWMCLRRFIIFLFILLLCLISLLVLLDYQGMLPVCPPLLGKSNTTTTSTGPC
KTCTMPAQGTSMFPSCCCTKPSDGNCTCIPIPSSWAFARFLWEWASVRFSWLSLLVPFVQWF
VGLSPTVWLSVIWMMWYWGPSLYNILSPFLPLLPISCCLWAYIGSATSNVSEDVRDNVSVDY
KQTQLCILGCAPAIGEHWAKGTACKSRPLSQGDCPPLELKNTVLEDGDMVDTGYGAMDFSTL
QDTKCEVPLDICQSICKYPDYLQMSADPYGDSMFFCLRREQLFARHFWNRAGTMGDTVPQSL
YIKGTGMRASPGSCVYSPS 3′

Chimeric fusion of a larger fragment of HPV18 consisting of the following sequence: 
5′
ATSNVSEDVRDNVSVDYKQTQLCILGCAPAIGEHWAKGTACKSRPLSQGDCPPLELKNTVLE
DGDMVDTGYGAMDFSTLQDTKCEVPLDICQSICKYPDYLQMSADPYGDSMFFCLRREQLFAR
HFWNRAGTMGDTVPQSLYIKGTGMRASPGSCVYSPS 3′
 and fused at its C-terminus to the N-terminus of the HbsAg to obtain the chimeric sequence of SEQ ID 12:

SEQ ID No.12:
MATSNVSEDVRDNVSVDYKQTQLCILGCAPAIGEHWAKGTACKSRPLSQGDCPPLELKNTVL
EDGDMVDTGYGAMDFSTLQDTKCEVPLDICQSICKYPDYLQMSADPYGDSMFFCLRREQLFA
RHFWNRAGTMGDTVPQSLYIKGTGMRASPGSCVYSPSGSENTTSGFLGPLLVLQAGFFLLTR
ILTIPQSLDSWWTSLNFLGGAPACPGQNSQSPTSNHSPTSCPPICPGYRWMCLRRFIIFLFI
LLLCLISLLVLLDYQGMLPVCPPLLGKSNTTTTSTGPCKTCTMPAQGTSMFPSCCCTKPSDG

NCTCIPIPSSWAFARFLWEWASVRFSWLSLLVPFVQWFVGLSPTVWLSVIWMMWYWGPSLYN
ILSPFLPLLPISCCLWAYI

### Strategy No. 7:

Chimeric fusion of the full length sequence of HPV16 L1 protein at its N-terminus to the signal sequence of 24 amino acids of the human serum albumin for secretory expression in pichia pastoris in order to obtain SEQ ID NO. 13

SEQ ID NO. 13:
MKWVTFISLLFLFSSAYSRGVFRRGSSLWLPSEATVYLPPVPVSKVVSTDEYVARTNIYYHAGTSRLLAVG
HPYFPIKKPNNNKILVPKVSGLQYRVFRIHLPDPNKFGFPDTSFYNPDTQRLVWACVGVEVGRGQPLGVGISGHPL
LNKLDDTENASAYAANAGVDNRECISMDYKQTQLCLIGCKPPIGEHWGKGSPCTNVAVNPGDCPPLELINTVIQDG
DMVDTGFGAMDFTTLQANKSEVPLDICTSICKYPDYIKMVSEPYGDSLFFYLRREQMFVRHLFNRAGAVGENVPDD
LYIKGSGSTANLASSNYFPTPSGSMVTSDAQIFNKPYWLQRAQGHNNGICWGNQLFVTVVDTTRSTNMSLCAAIST
SETTYKNTNFKEYLRHGEEYDLQFIFQLCKITLTADVMTYIHSMNSTILEDWNFGLQPPPGGTLEDTYRFVTSQAI
ACQKHTPPAPKEDPLKKYTFWEVNLKEKFSADLDQFPLGRKFLLQAGLKAKPKFTLGKRKATPTTSSTSTTAKRKK
RKL

### SEQ ID NO.14:

Chimeric fusion of the full length sequence of HPV16 L2 protein at its N-terminus to the signal sequence of 24 amino acids of the human serum albumin for secretory expression in pichia pastoris in order to obtain SEQ ID NO. 14

MKWVTFISLLFLFSSAYSRGVFRRGSRHKRSAKRTKRASATQLYKTCKQAGTCPPDIIPKVEG
KTIADQILQYGSMGVFFGGLGIGTGSGTGGRTGYIPLGTRPPTATDTLAPVRPPLTVDPVGPSDP
SIVSLVEETSFIDAGAPTPVPSIPPDVSGFSITTSTDTTPAILDINNTVTTVTTHNNPTFTDPSV
LQPPTPAETGGHFTLSSSTISTHNYEEIPMDTFIVSTNPNTVTSSTPIPGSRPVARLGLYSRTTQ
QVKVVDPAFVTTPTKLITYDNPAYEGIDVDNTLYFSSNDNSINIAPDPDFLDIVALHRPALTSR
RTGIRYSRIGNKQTLRTRSGKSIGAKVHYYYDFSTIDPAEEIELQTITPSTYTTTSHAAS
PTSINNGLYDIYADDFITDTSTTPVPSVPSTSLSGYIPANTTIPFGGAYNIPLVSGPDIP
INITDQAPSLIPIVPGSPQYTIIADAGDFYLHPSYYMLRKRRKRLPYFFSDVSLAA

### SEQ ID No. 15:

Chimeric fusion of the full length sequence of HPV18 L1 protein at its N-terminus to the signal sequence of 24 amino acids of the human serum albumin for secretory expression in pichia pastoris in order to obtain SEQ ID NO. 15:

MKWVTFISLLFLFSSAYSRGVFRRGSLWRPSDNTVYLPPPSVARVVNTDDYVTRTSIFYHAG
SSRLLTVGNPYFRVPAGGGNKQDIPKVSAYQYRVFRVQLPDPNKFGLPDNSIYNPETQRLVW
ACAGVEIGRGQPLGVGLSGHPFYNKLDDTESSHAATSNVSEDVRDNVSVDYKQTQLCILGCA
PAIGEHWAKGTACKSRPLSQGDCPPLELKNTVLEDGDMVDTGYGAMDFSTLQDTKCEVPLDI
CQSICKYPDYLQMSADPYGDSMFFCLRREQLFARHFWNRAGTMGDTVPQSLYIKGTGMRASP
GSCVYSPSPSGSIVTSDSQLFNKPYWLHKAQGHNNGVCWHNQLFVTVVDTTRSTNLTICAST
QSPVPGQYDATKFKQYSRHVEEYDLQFIFQLCTITLTADVMSYIHSMNSSILEDWNFGVPPP
PTTSLVDTYRFVQSVAITCQKDAAPAENKDPYDKLKFWNVDLKEKFSLDLDQYPLGRKFLVQ
AGLRRKPTIGPRKRSAPSATTSSKPAKRVRVRARK

### 2. Example 2: Gene cloning and the construction of HbsAg-HPV antigen chimeras:

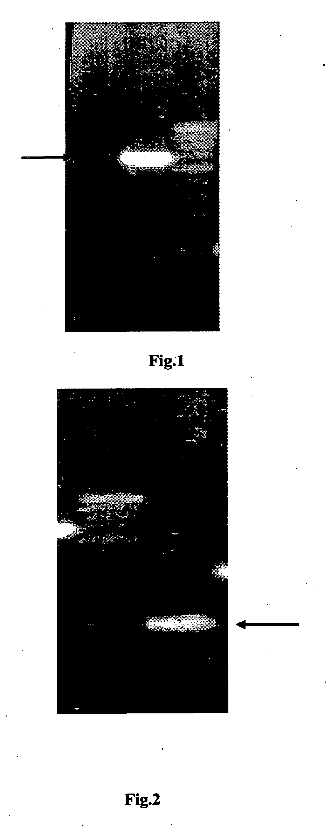

a) Hepatitis B gene encoding the full length HbsAg protein was PCR amplified with N-terminal and C-terminal primers containing EcoR1 and BamH1 restriction sites respectively to obtain a ∼ 700 bp PCR fragment (See Fig.1). The primers used for PCR amplification of the HbsAg gene are:
   HBNTERM:
      5' CACGAATTCACCATGGAGAACACAACATCAGG 3'
   HBCTERM:
      5' TCAGGATCCAATGTATGCCCAAAGACAACAGG 3'

The conditions for PCR amplification were denaturation at 94°C for 45 seconds, annealing at 65°C for 40 seconds and extension at 72°C for 1.5 minutes for 30 cycles followed by final extension at 72°C for 10 minutes. 10 ng of the plasmid DNA containing HbsAg gene cloned in pBluescript SKII+ was used as the template for PCR reaction. The reaction consisted of 1 x Taq DNA polymerase buffer, 0.25 mM dNTPS, 20 picomoles of each primer and 1.5 U of Taq DNA polymerse. All the reagents were from Bangalore Genei. The PCR fragment was gel purified and digested with BamH1. BamH1 digested HbsAg PCR fragment was used for ligation with the various HPV antigens inorder to obtain the chimeric constructs. For construction of the chimeric constructs which carried the HbsAg gene at the C terminus and the HPV antigens at the N-terminus, the HbsAg was PCR amplified with a C-term primer that contained a Not1 site and the N-term primer that contained the BamH1 site. In the constructs used for PCR amplification where the HPV antigens are at the N-term and HbsAg are at the C-term, the PCR primers for HPV antigens contained an EcoR1 site at their N-terminus and a BamH1 site at their C-terminus. In both cases, chimeric fusion the HbsAg gene and the HPV gene fragments were made through ligation at the BamH1 end of both the sequences. The BamH1 site introduced two amino acids 'GS' (Glycine-Serine) at the junction of the HbsAg-HPV sequences. All chimeric fusions described in this disclosure (SEQ ID No.1 to SEQ ID No.15) has been carried out at the nucleotide level using polymerase chain reaction (PCR) and ligation of the PCR amplified genes.

The following HPV gene fragments were amplified by PCR for ligation:

### Cloning of the multiepitope sequence:

EYMLDLQPETTEEDEIDGPAGQAEPDRAHYNIDEIDGVNHQHL - 43 amino acids (129 nucleotides). The synthetic gene fragment encoding the above sequence was synthesized at GenScript Corporation. The gene fragment was amplified with the following primers that introduced a BamH1 site at N'terminus and a Not 1 site at the C-terminus. PCR reaction was denaturation at 94°C for 45 seconds, annealing at 62°C for 45 seconds, and extension at 72°C for 30 seconds for 33 cycles inorder to obtain a ∼ 140 bp PCR fragment containing 129 bp of the epitope sequence.
MENTERM primer:
   5' ATAGGATCCGAGTACATGTTGGATTTG 3'
MECTERM primer:
   5' ATCGCGGCCGCTTATCACAAATGTTGGTGGTTG 3'

The 129 bp PCR fragment (See Fig.2) using the above primers was gel purified and digested with BamH1. The BamH1 digested PCR fragment was ligated to BamH1 digested HbsAg gene overnight using T4 DNA ligase inorder to obtain the chimeric fragment of ∼ 840 bp whose translated sequence is designated as **SEQ ID NO.1.** The ligated fragment of the correct size (∼ 840 bp) was gel purified, digested with EcoR1 and Not 1 for cloning into the yeast vector pPIC3.5K. In order to generate SEQ ID NO.2, wherein the multiepitope sequence is ligated at the 5' end of the HbsAg gene, primers of similar sequences were made except that C-terminus primer carried a BamH1 site instead of Not1 site and in the N-term primer, the BamH1 site was replaced with EcoR1. The PCR product of ∼140 bp was digested with BamH1, and ligated with BamH1 digested HbsAg overnight with T4 DNA ligase. The ligated ∼ 840 bp fragment designated as **SEQ ID NO.2** was gel purified, digested with EcoR1 and Not 1 for cloning into the yeast expression vector, pPIC3.5K.

### Chimeric fusion of the of the HPV16 L2 fragment at the C-terminus of HbsAg.

The N-terminal region of the minor capsid protein L2 was fused to the HbsAg at the C-terminus of the HbsAg. HPV16 L2 gene was synthesized at GenScript Corp. The regions of L2 amplified correspond to amino acids 50 - 220 (510 bp) and ligated at the C-terminus of the HbsAg to generate the **SEQ ID NO.3.** For ligation of the L2 gene fragment to the C-terminus of the HbsAg gene, the gene encoding the L2 protein was PCR amplified with a similar N-term primer containing BamH1 site and a C-term primer containing Not1 site. The PCR amplified ∼ 530 bp fragment was digested with BamH1 and ligated with BamH1 digested HbsAg that carried a BamH1 site at its C-terminus. The ligated ∼ 1230 bp fragment was gel purified and digested with EcoR1 and Not1 for cloning into the yeast vector pPIC3.5K.

For generation of the HPV-HbsAg chimeric construct, the L2 gene (amino acid position 50 - 220bp) was amplified with primers of similar sequence except that the N-terminus primer carried a EcoR1 site instead of BamH1 and the C-term primer contained a BamH1 site instead of Not1. The PCR amplification, digestion was as described above to obtain **SEQ ID NO.4.**

### Chimeric fusion of the nested deletions of the HPV16 L2 fragment at the C-terminus of HbsAg.

The N-terminal region of the minor capsid protein L2 was fused to the HbsAg at the C-terminus of the HbsAg. The regions of L2 amplified are 100 - 220 amino acids (120 aa; 360 bp). The primers used for PCR amplification of L2 gene fragment are:
6P2CFNTERM:
   5' ACCGGATCCGATCCATCTATCGTTTC 3'
6P2CFCTERM:
   5' ATCGCGGCCGCTTATCATCTTGAACCTGGAATTG 3'

PCR reaction was carried out in a 50 µL volume containing 1x Taq DNA polymerase buffer, 0.25 mM dNTPs, and 20 picomoles of each primer and 1.5 U Taq DNA polymerase. The PCR conditions were denaturation at 94°C for 45 seconds, 64°C for 40 seconds and extension at 72°C for 1 min for 30 cycles.

The ∼ 380 bp PCR fragment containing 360 bp of L2 sequence was gel purified, digested with BamH1 and ligated to BamH1 digested HbsAg gene to generate the HbsAg-HPV chimera of ∼ 1060 bp designated as **SEQ ID No. 5.** For the generation of the HPV-HbsAg chimeric sequence wherein the L2 gene fragment is ligated to the N'terminus of the HbsAg gene, the N-term and C-term primers for L2 contained EcoR1 and BamH1 restriction sites. The ligation was carried out the with BamH1 digested L2 gene fragment and the BamH1 digested HbsAg to generate the 1060 bp of the chimeric sequence designated as **SEQ ID NO.6.**

### Generation of the chimeric sequence with L1 region of HPV16 and HbsAg:

Generation of the chimeric sequence consisting of 33 amino acids derived from the L1 region of the HPV16 protein (99 bp) of the sequence: NTVIQDGDMVDTGFGAMDFTTLQANKSEVPLDI with HbsAg gene. The specified region of the HPV16 L1 gene (gene synthesized at GenScript Corporation) was amplified by PCR to obtain a∼ 120 bp PCR fragment containing the 99 bp of the above mentioned sequence was amplified using the following PCR primers:
6P1CFNTERM:
   5'ATCGGATCCAACACCGTTATCCAAGACGG3'
6P1CFCTERM:
   5' ACTGCGGCCGCTTATCAAATATCCAATGGAACTTC 3'

The PCR reaction was carried out in 50 µL reaction volume containing 1x Taq DNA polymerase buffer, 0.25 mM dNTPs, 20 picomoles of each primer and 1.5 U Taq DNA polymerase (all reagents obtained from Bangalore Genei). The PCR cycle consisted of denaturation at 94°C for 45 seconds, annealing at 65°C for 40 seconds, and extension at 72°C for 45 seconds for 30 cycles. The ∼ 120 bp PCR fragment was gel purified, digested with BamH1 and ligated to the BamH1 digested HbsAg overnight with T4 DNA ligase to generate a chimeric sequence of ∼ 810 bp designated as **SEQ ID No.7** in which the HPV16 L1 fragment is C-terminal to the HbsAg gene. Inorder to generate a HPV-HbsAg when the above mentioned fragment is ligated at the N-terminus of HbsAg gene, the HPV16L1 fragment was PCR amplified with primers of similar sequence containing a EcoR1 site in the N-terminal primer and a BamH1 sequence at the C-term primer instead of Not1. The PCR fragment of similar size is digested with BamH1 and ligated to the BamH1 digested HbsAg as described above to generate the sequence designated as **SEQ ID No.8.**

### Generation of the chimeric sequence with L1 region of HPV18 and HbsAg:

Generation of the chimeric sequence of HPV18 L1 protein (39 amino acids; 117 bp) of the following sequence PPLELKNTVLEDGDMVDTGYGAMDFSTLQDTKCEVPLDI ligated to the C-temrinus of the HbsAg sequence by PCR. The synthetic HPV18 L1 gene was codon optimized for expression in *Pichia pastoris* and was synthesized at GenScript Corporation. The HPV18 L1 sequence specified above was PCR amplified with the with the following PCR primers:
8PICFNTERM:
   5' GATGGATCCCCTCCTTTGGAATTGAAG 3'
8P1CFCTERM:
   5' AGTGCGGCCGCTTATCAGTAATCTGGATATTTGC 3'

The PCR reaction was carried out in a 50 µL volume and contained 1 x Taq DNA polymerase buffer, 0.25 mM dNTPs, 20 pico moles of each primer and 1.5 U Taq DNA polymerase. The PCR reaction consisted of denaturation at 94°C for 45 seconds, annealing at 65°C for 30 seconds and extension at 72°C for 40 seconds for 30 cycles. The ∼ 135 bp PCR fragment was gel purified, digested with BamH1 and ligated to BamH1 digested HbsAg to obtain a HbsAg -HPV chimeric sequence where the HPV18L1 sequence is ligated to the C-terminus of HbsAg to obtained the **SEQ ID No.9.** For generation of the HPV-HbsAg chimeric sequence, the PCR primers contained a EcoR1 site in the N-term primer and a BamH1 site in the C-term primer. After digestion with BamH1, the fragment was ligated to the HbsAg that contained a BamH1 at the N'terminal end to generate the **SEQ ID No.10.** Both the ligated PCR fragments were digested EcoR1 and Not 1 for cloning into the yeast expression vector pPIC3.5K.

### Generation of Chimeric Sequence of larger fragment of HPV18 L1 with HbsAg:

Chimeric sequence of a larger fragment of HPV18 L1 consisting of the following sequence 
5′ATSNVSEDVRDNVSVDYKQTQLCILGCAPAIGEHWAKGTACKSRPLSQGDCPPLELKNTV
LEDGDMVDTGYGAMDFSTLQDTKCEVPLDICQSICKYPDYLQMSADPYGDSMFFCLRREQLF
ARHFWNRAGTMGDTVPQSLYIKGTGMRASPGSCVYSPS 3′
 was ligated to the C-terminus of the HbsAg by PCR. The L1 fragment was PCR amplified with the following PCR primers:
8P1CFLFNTERM:
   5' TCTGGATCCGCTACATCTAATGTCTC 3'
8P1CFLFCTERM:
   5' ATTGCGGCCGCTTATCAGGATGGACTGTAAACG 3'

The 50 µL PCR reaction consisted of 1 x Taq DNA polymerase buffer, 0.25 mM dNTPs, 20 picomoles each of the N- and C-term primers and 1.5 U of Taq DNA polymerase. The PCR reaction consisted of 1 x Taq DNA polymerase buffer, 0.25 mM dNTPs, 20 picomoles of each primer and 1.5 U Taq DNA polymerase. The PCR cycle consisted of denaturation at 94°C for 30 seconds, annealing at 64°C for 40 seconds and extension at 72°C for 1 min for 30 cycles. The PCR fragment was gel purified, digested with BamH1 and ligated to the C-terminus of BamH1 digested HbsAg to obtain the chimeric fragment of **SEQ ID No. 11.** For the ligation of the above mentioned HPV18 L1 fragment to the N'terminus of the HbsAg sequence, the primers carried a EcoR1 site for the N-term primer and a BamH1 site for the C-term primer. The BamH1 digested fragment was ligated to the BamH1 digested HbsAg to obtain the chimeric sequence in which the HPV18 L1 fragment is at the N-terminus of the HbsAg gene to obtain the **SEQ ID No. 12.** Both the above chimeric sequences were digested with EcoR1 and Not1 and ligated to the EcoR1 and Not1 digested yeast expression vector pPIC3.5 K for cloning in *Pichia pastoris.*

### Generation of chimeric sequences of HPV antigens with the signal sequence of Human Serum albumin gene for secretory expression of HPV antigens in Pichia pastoris:

The 24 amino acid signal sequence of human serum albumin gene was PCR amplified with the following primers:
HASSNTERM:
   5' CCTGAATTCACCATGAAGTGGGTAACCTTTATTTC 3'
HASSCTERM:
   5' ATTGGATCCTCGACGAAACACACCCCTG 3'
to obtain a 72 bp fragment that was gel purified, digested with BamH1 and ligated to the 5' end of the full length genes of HPV16 L1, HPV16L2 and HPV18 L1 that carried a BamH1 site at the 5'end to obtain the sequences SEQ ID No. 13, SEQ ID NO.14 and SEQ ID No.15 respectively.

### Example 3: Cloning and Expression in Pichia Pastoris:

All the chimeric sequences were verified by sequencing both the strands of the DNA for correctness of the chimeric sequence and the reading frame before proceeding with yeast transformation. All the chimeric sequences mentioned in the previous sections after digestion with EcoR1 and Not1 were gel purified and ligated with EcoR1 and Not1 digested pPIC3.5 K vector (Invitrogen Corporation, Carlsbad, USA) and ligated overnight with T4 DNA ligase. The ligation mix was transformed in competent E.coli DH5a cells and plated in LB agar plate containing 100 µg/mL ampicillin. Plasmid DNA was isolated from transformed clones for each of the constructs in large scale, purified and digested with Bgl II to linearize the plasmid for yeast transformation.

Essentially the linearized plasmid DNA encoding the chimeric sequences from SEQ ID No.1 to SEQ ID No.15 were transformed into *Pichia Pastoris* GS115 as per the manufacturers (Invitrogen) protocols and as outlined in their manual. All the chimeric sequences were cloned into the AOX1 locus and expressed under the AOX1 promoter by methanol induction. The cloning, screening, isolation of the recombinant *Pichia* strains and induction of the cloned gene with methanol were carried out as per the User's manual "A Manual of Methods for Expression of Recombinant Proteins in Pichia pastoris" Version M Jan 2002, of Pichia Expression Kit, Catalog # K1710-01, Invitrogen Corporation, Carlsbad, USA).

### Example 4: Purification of Chimeric HbsAg-HPV antigens:

The cells after induction with methanol were harvested, and lysed by sonication and /or with glass beads. The lysate was treated with PEG6000 to a concentration of 7.5 % and NaCl upto 3%. The cell suspension was centrifuged at 8000 x g for 10 min. The supernatant was precipitated with salt treatment (proprietary mix) and precipitated. The protein was eluted from the salt precipitate with Tris-HCl buffer, pH 8.5. The elute was concentrated and diafiltered with 50 mM Tris-HCl, pH 8.5 and loaded on a cation column equilibrated with the same buffer. The protein was eluted with NaCl of different concentration for the different antigenic proteins. The purity of the preparation was checked on 12% SDS-PAGE with silver staining. The correct molecular sizes of the various chimeric proteins were confirmed subsequently by Western blot.

### Example 5: ELISA of the HPV-HbsAg chimeric proteins:

The expression of the chimeric proteins were detected by ELISA for the hepatitis B small antigen content by MONOLISA Anti-Hbs3.0 kit (product # 72400; Bio-Rad) exactly as the per the protocol outlined in the kit manual and expressed in ng/mL. 100 µL of the cell lysate after induction was used for the estimation of HbsAg content. The estimation of HPV antigens in the chimeric proteins in the cell lysate after induction was also carried out by ELISA. 1 µg of the purified proteins in 200 µL were coated on a 96-well microtiter plate in the presence of coating buffer (carbonate buffer, pH 9.6) and incubated overnight at 4°C. The contents of the wells were discarded after tapping the plate on a paper towel to remove residual buffer in the wells. The wells were blocked with 225 µL/well of blocking solution (1 x PBST containing 0.5% BSA) and incubated at 37°C for 1 hour.

The wells were washed three times with wash buffer (200 µL/well, 30 seconds per wash). The plates were tapped dry on paper towel after each wash to remove residual buffer from the wells. The wells were washed in 1x PBST (Phosphate buffer containing 0.5% tween 20) five times to remove the unbound proteins. Antibody for full length HPV6L1, HPV16 L2 and HPV18 L1 raised in rabbit was used as the primary antibody. After addition of the primary antibody at 1:1000 dilution, the plates were incubated at room temperature for one hour with shaking. The contents of the wells were discarded and washed five times with wash buffer containing 1xPBST. The secondary antibody anti-rabbit IgG-HRP conjugate was added at 1:2000 dilution in 10 mM phosphate buffered saline and incubated at room temperature for one hour with shaking. The contents of the wells were discarded and the plates were tapped on dry paper towel to remove residual buffer in the wells. 200 µL of freshly prepared substrate solution OPD (Orthophenylene diamine dihydrochloride) and hydrogen peroxide was added per well and incubated at room temperature for 30 min. The reaction was stopped by adding 75 µL of the stopping solution and the absorbance at 490 nm was read in the ELISA reader.

### Example 6: Western blotting

The chimeric proteins as described in the previous sections were separated on 12% SDS-PAGE gel, electroblotted on PVDF (Hybond-P, GE Healthcare) membrane and blocked in a solution containing 10 mM phosphate buffer, pH7.4 containing 3% skimmed milk powder. After overnight blocking with the blocking solution, the blocking solution was discarded and rinsed 3x 5 minutes in PBST. The blot was incubated at 37°C with 1: 500 dilution of the primary rabbit-antisera raised with a commercially available tetravalent vaccine. After incubating with primary antibody for one hour, the blot was washed five times and incubated at 1:2000 dilution in PBST of the secondary antibody i.e. anti-rabbit IgG HRPO conjugate and incubated at RT for one hour with shaking. The substrate diaminobenzidene (DAB) and hydrogen peroxide was added and color developed till the required color intensity was achieved. The reaction was stopped by discarding the substrate solution and rinsing with WFI.

Western blot detected HPV16 L1 and HPV18 L1 in the chimeric sequences with the antisera raised against the commercially available tetravalent HPV vaccine.

### Example 7: Immunogenicity of the Chimeric proteins:

10 BALB/C mice (15-20g) in each test group were immunized with 50 µg of the purified protein of each of the chimeric antigens absorbed in aluminum hydroxide gel and administered intramuscularly. A booster dose was given 21 days after the 1^{st} dose and a second dose was administered after 45 days after the 1^{st} dose. The antibody titer was measured by indirect ELISA for all clones containing SEQ ID Nos. 3 -12 as outlined in the previous section. The antibody titer was estimated for both the HbsAg and the HPV antigens in mice immunized with the chimeric antigens. The primary antibody for estimation of HPV16L1 and HPV18 L1 was the rabbit antisera raised against the commercially available tetravalent vaccine except for clones expressing HPV16 L2 protein which was measured with HPV16L2 rabbit antisera raised against the purified HPV16L2 protein. The antibody titer against the HPV antigens was measured by indirect ELISA in the pooled sera from each group of mice. The secondary antibody was rabbit anti-IgG HRPO conjugate. The experiment was repeated where the equivalent amount of antigens in algel were administered with 50 µg of CpG oligonucleotides per dose. The Antibody titer in all the mice was estimated at 60-65 days after the administration of the initial dose of the antigen. For the estimation of antibody titer against the HbsAg in the immunized mice, the estimations were carried out with Monolisa Anti HBs 3.0 Kit from Bio-Rad laboratories exactly as per the manufacturer's instructions. The values were recorded at 490 nm and plotted.

### References:

Christensen N., Reed C., Cladel N., Han R and Kreider J. (1996): Immunization with viruslike particles induces long-term protection of rabbits against challenge with cottontail rabbit papillomavirus. Journal of Virology 70, 960-965.
Kirnbauer R., Chandrachud L., O'Neill B., Wagner E., Grindlay G., Armstrong A., McGarvie G., Schiller J., Lowry D. and Camp M. (1996): Virus-like particles of bovine papillomavirus type-4 in prophylactic and therapeutic immunization. Virology 219, 37-44.
Rose R., White W., Maolin L., Suzich J., Lane C. and Garcea R. (1998): Human papillomavirus type 11 recombinant L1 capsomeres induce virus neutralizing antibodies Journal of Virology 72, 6151-6154.
Walboomers JM, Jacobs MV, Manos MM, Bosch FX, Kummer JA, Shah KV, Snijders PJ, Peto J, Meijer CJ, Munoz N. Human papillomavirus is a necessary cause of invasive cervical cancer worldwide. J Pathol 1999; 189: 12-9.
Bosch FX, de Sanjose' S. Chapter 1: Human papillomavirus and cervical cancer burden and assessment of causality. J Natl Cancer Inst Monogr 2003;31:3 - 13.
Schlecht NF, Platt RW, Duarte-Franco E, et al. Human papillomavirus infection and time to progression and regression of cervical intraepithelial neoplasia. J Natl Cancer Inst 2003; 95:1336 - 43.
Richardson H, Kelsall G, Tellier P, et al. The natural history of type-specific human papillomavirus infections in female university students. Cancer Epidemiol Biomarkers Prev 2003, 12:485 - 90.
Londesborough P, Ho L, Terry G, Cuzick J, Wheeler C, Singer A. Human papillomavirus genotype as a predictor of persistence and development of high-grade lesions in women with minor cervical abnormalities. Int J Cancer 1996, 69:364 - 8.
Clifford GM, Smith JS, Plummer M, Munoz N and Francheschi S. Human papillomavirus types in invasive cervical cancer worldwide: a meta-analysis. British J Cancer 2003; 88:63-73.
Clifford GM, Smith JS, Plummer M, Munoz N and Francheschi S. Human papillomavirus types in invasive cervical cancer worldwide: a meta-analysis. British J Cancer 2003; 88:63-73.
Pereira R, Hitzeroth II, Rybicki EP. Insights into the role and function of L2, the minor capsid protein of papillomavirusesArch Virol. 2009; 154(2): 187-97).
Kanda T, Kondo K. Development of an HPV vaccine for a broad spectrum of high-risk typesHum Vaccin. 2009 Jan-Feb;5(1):43-5;

## Claims

1. An antigenic formulation consisting of:
chimeric fusion antigens of HPV antigens with Hepatitis B surface antigen (HbsAg); and
an adjuvant;
wherein the HPV-HbsAg chimeric fusion antigens are selected from the group consisting of SEQ ID No. 1 and SEQ ID No. 2.

2. The formulation according to claim 1, wherein the adjuvant is selected from at least one of the following: aluminium hydroxide, aluminium phosphate, monophosphoryl lipid A, CpG oligonucleotides, or cytokines.

3. A method for preparing the HPV-HbsAg chimeric fusions of claim 1, wherein the method comprises the step of expressing the chimeric fusions in either prokaryotic or eukaryotic expression systems as intracellular or secretory proteins.

4. A pharmaceutical composition for use in the prophylaxis and treatment against Human papillomavirus (HPV) and Hepatitis B infection in humans, said composition comprising: an antigenic formulation according to any of claims 1 or 2, and a pharmaceutically acceptable carrier, wherein the chimeric fusion antigens are used in the range of 10 µg to 100 µg of the protein per dose.

## Patentansprüche

1. Antigene Zubereitung, bestehend aus:
chimären Fusionsantigenen von HPV-Antigenen mit Hepatitis-B-Oberflächenantigen (HbsAg); und
einem Adjuvans;
wobei die chimären HPV-HbsAg-Fusionsantigene aus der Gruppe ausgewählt sind, die aus SEQ ID Nr. 1 und SEQ ID Nr. 2 besteht.

2. Zubereitung gemäß Anspruch 1, wobei das Adjuvans aus wenigstens einem der folgenden ausgewählt ist: Aluminiumhydroxid, Aluminiumphosphat, Monophosphoryl-Lipid-A, CpG-Oligonucleotiden oder Cytokinen.

3. Verfahren zur Herstellung der chimären HPV-HbsAg-Fusionen gemäß Anspruch 1, wobei das Verfahren den Schritt des Exprimierens der chimären Fusionen in entweder prokaryontischen oder eukaryontischen Expressionssystemen als intrazelluläre oder sekretorische Proteine umfasst.

4. Pharmazeutische Zusammensetzung zur Verwendung bei der Prophylaxe und Behandlung von Humanpapillomvirus(HPV)- und Hepatitis-B-Infektion beim Menschen, wobei die Zusammensetzung umfasst: eine antigene Zubereitung gemäß einem der Ansprüche 1 oder 2 und einen pharmazeutisch annehmbaren Träger, wobei die chimären Fusionsantigene im Bereich von 10 µg bis 100 µg des Proteins pro Dosis verwendet werden.

## Revendications

1. Formulation antigénique consistant en
des antigènes de fusion chimériques constitués d'antigènes HPV avec l'antigène de surface de l'hépatite B (Ag HBs), et
un adjuvant,
dans laquelle les antigènes de fusion chimériques HPV-(Ag HBs) sont choisis dans le groupe consistant en SEQ ID n° 1 et SEQ ID n° 2.

2. Formulation selon la revendication 1, dans laquelle ledit adjuvant est choisi parmi au moins un des adjuvants suivants : hydroxyde d'aluminium, phosphate d'aluminium, monophosphoryl-lipide A, oligonucléotides CpG, ou cytokines.

3. Procédé pour préparer lesdites fusions chimériques HPV-(Ag HBs) selon la revendication 1, dans lequel le procédé comprend l'étape consistant à exprimer les fusions chimériques dans des systèmes d'expression soit procaryotes soit eucaryotes comme protéines intracellulaires ou sécrétoires.

4. Composition pharmaceutique à utiliser dans la prophylaxie et le traitement d'infections avec le papillomavirus humain (pvh) et hépatite B chez l'homme, ladite composition comprenant : une formulation antigénique selon l'une quelconque des revendications 1 ou 2, et une véhicule pharmaceutiquement acceptable, dans laquelle les antigènes de fusion chimériques sont utilisés dans une gamme de 10 µg à 100 µg de la protéine par dose.
